Europäisches Patentamt

European Patent Office

Office européen des brevets

(11) Numéro de publication: **0 278 186**
**A1**

## (12) DEMANDE DE BREVET EUROPEEN

(21) Numéro de dépôt: 87400322.1

(22) Date de dépôt: **13.02.87**

(51) Int. Cl.⁴: **A61M 5/32**

(43) Date de publication de la demande:
**17.08.88 Bulletin 88/33**

(84) Etats contractants désignés:
**AT BE CH DE ES GB GR IT LI LU NL SE**

(71) Demandeur: **SOFIC Société Anonyme**
**1 Rue de la Vanne**
**F-81200 Mazamet(FR)**

(72) Inventeur: **Granier, Patrick M.**
**41, Rue de la République**
**F-81200 Mazamet(FR)**

(74) Mandataire: **Morelle, Guy Georges Alain**
**SC Morelle & Bardou 1, Avenue de Ranguell**
**F-31400 Toulouse(FR)**

(54) **Aiguille pour seringue.**

(57) La présente invention concerne les aiguilles pour seringues.

L'aiguille selon l'invention comporte un tube (1) cylindrique fin traversé par un canal (3) suivant l'axe (2), l'extrémité (6) du tube est taillée suivant un plan incliné (10) pour définir une surface en biseau (9), ledit canal débouchant par au moins un orifice (7) sur la paroi latérale de l'extrémité (6) du tube et au niveau d'une génératrice (12) de celui-ci, passant sensiblement par la perpendiculaire (13) au plan incliné (10) élevée au point de concours de la surface en biseau (9) et de l'axe (2) du tube.

L'aiguille selon l'invention trouve une application avantageuse dans les domaines notamment médical, dentaire et vétérinaire.

fig.1

EP 0 278 186 A1

## AIGUILLE POUR SERINGUE

La présente invention concerne les aiguilles pour seringues, pour des applications dentaires, médicales et/ou vétérinaires.

On connaît un grand nombre de modèles de seringues du type médical, constituées essentiellement par un tube généralement long et fin terminé par une pointe et comportant en son centre un canal qui débouche à l'extrémité du tube. Quand une telle aiguille pénètre dans une certaine qualité de chair, notamment les gencives ou les tissus dentinaires, l'orifice d'extrémité peut être obturé par des particules du type osseux ou autre. Pour éviter cet inconvénient, on a réalisé sur la paroi latérale cylindrique des tubes fins, des orifices qui peuvent ainsi, théoriquement, échapper à une certaine obturation. Cependant, du fait de la forme en pointe de ces tubes, les éléments ou particules sont repoussés tout autour de la paroi latérale du tube et viennent encore, quelquefois, obturer ces orifices.

Aussi, la présente invention a-t-elle pour but de pallier cet inconvénient dans la plupart des cas.

Plus précisément, la présente invention a pour objet une aiguille pour seringue, pour applications médicales, dentaires ou vétérinaires ou analogues, constituée d'un tube relativement fin et cylindrique comportant en son centre un canal, ledit tube comportant à une extrémité une pointe de pénétration, ledit canal débouchant sensiblement au niveau de cette pointe de pénétration, caractérisée en ce que ladite pointe de pénétration est constituée par une surface sensiblement plane, unique, suivant un premier plan incliné sur l'axe du tube cylindrique formant ainsi un biseau, ledit canal débouchant par au moins un orifice sur la paroi latérale dudit tube cylindrique à un niveau situé sensiblement à proximité dudit plan incliné, une génératrice solide dudit tube passant par la perpendiculaire audit plan incliné et élevée au point de concours de l'axe dudit tube cylindrique et dudit plan incliné.

D'autres caractéristiques et avantages de la présente invention apparaîtront au cours de la description suivante donnée en regard des dessins annexés à titre illustratif, mais nullement limitatif, dans lesquels:

-les Figures 1 et 2 représentent, respectivement selon des vues en coupe et en perspective, un mode de réalisation d'une aiguille selon l'invention et,

-la Figure 3 représente un dessin explicatif faisant apparaître les avantages d'une aiguille selon l'invention.

Etant donné que l'ensemble de ces trois Figures représente un même mode de réalisation d'une aiguille selon l'invention, il est bien évident que les mêmes références y désignent les mêmes éléments et que, pour une meilleure compréhension, il sera nécessaire de se reporter aux Figures sur lesquelles apparaissent les références citées.

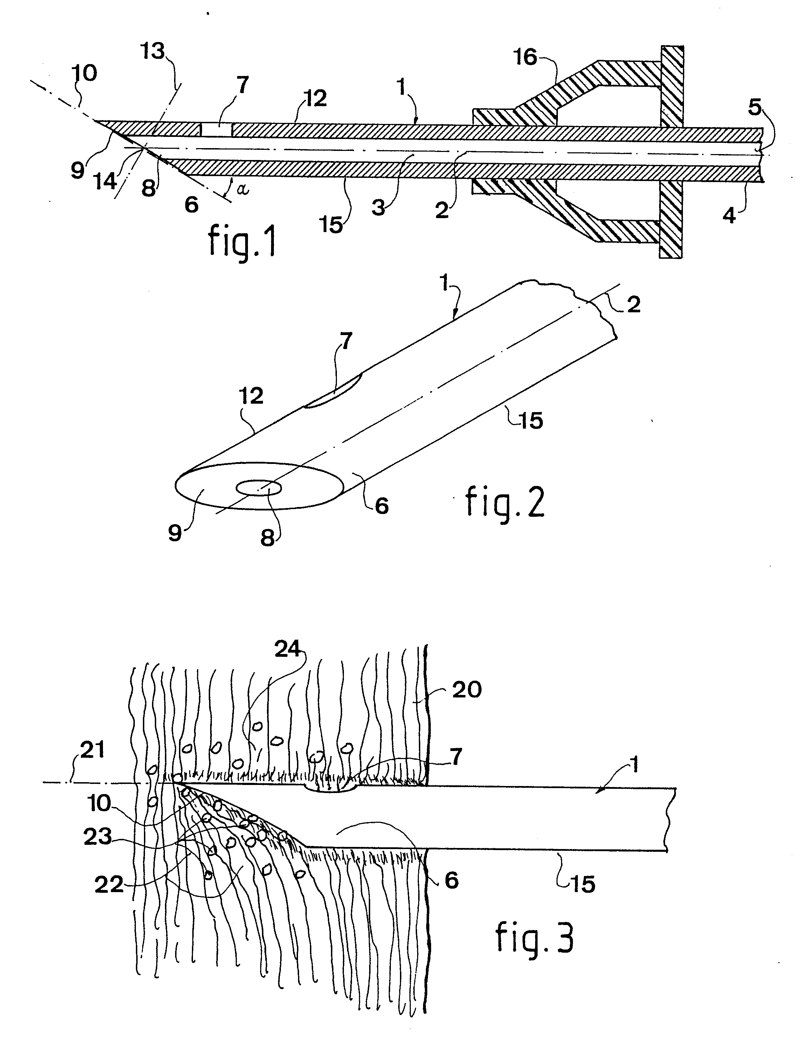

L'aiguille selon l'invention comporte essentiellement un tube généralement fin et cylindrique de révolution 1, réalisé, par exemple, en un matériau noble comme de l'acier inoxydable. Ce tube comporte en son centre, suivant l'axe 2, un canal central 3 débouchant à une extrémité 4 par un orifice 5 et, à l'autre extrémité 6, par certaines ouvertures 7 et 8. Pour permettre la pénétration dans les chairs, l'extrémité 6 du tube 1 est formée en pointe par un pan coupé 9 constitué par une surface qui peut être légèrement sphérique, avantageusement concave, comme représenté sur la Figure 1. De ce fait, l'extrémité 6 du tube 1 est limitée par un plan 10 faisant avec l'axe 2 un angle déterminé $\alpha$.

Avantageusement, le canal central 3 peut déboucher par l'orifice 8 situé au niveau du plan coupé 10, mais essentiellement par un orifice 7. Cet orifice 7 est situé, selon une caractéristique de la présente invention, au niveau d'une génératrice 12 du cylindre 1, cette génératrice passant par la perpendiculaire 13 élevée au point 14 de concours de l'axe 2 du cylindre 1 et du plan coupé 10, la génératrice considérée étant celle du cylindre solide 1, et non pas celle, 15, qui est coupée dans l'air, opposée à la précédente.

Bien évidemment on peut compléter la présence de cet orifice 7 par d'autres orifices, comme par exemple l'orifice 8 qui est, dans ce mode de réalisation, situé dans le plan coupé 9 et sur l'axe 2 du tube 1, mais qui pourrait éventuellement être bouché, le canal 3 ne comportant alors que l'orifice 7. Cependant, d'autres orifices peuvent être ouverts sur la périphérie de la portion d'extrémité 6 du tube 1, pour permettre une injection et une diffusion périphérique du liquide injecté, l'ouverture 7 étant néanmoins toujours présente pour assurer la sécurité de l'injection.

A l'aiguille telle que représentée, peuvent bien sûr être associés des moyens de fixation 16 à la seringue et qui sont schématiquement illustrés. Ces moyens étant bien connus en eux-mêmes, ils ne seront pas plus amplement décrits ici.

La Figure 3 représente une application d'une telle aiguille pénétrant dans les chairs 20 par son extrémité 6 pointue. On voit que, du fait que l'extrémité 6 du tube 1 est constituée d'une surface sensiblement plane ou légèrement sphérique 9 en biseau, celle-ci pénètre dans les chairs 20 suivant une direction 21 qui est définie par la génératrice 12 définie précédemment et les chairs 22, repoussées et tassées, ne le sont ainsi que d'un côté, c'est à dire du côté de la génératrice 15. Ceci a alors pour résultat de comprimer ces chairs 22 et de déplacer uniquement d'un côté l'ensemble des particules solides, osseuses ou analogues 23, ce qui évite qu'elles ne soient tassées, et en plus grand nombre, sur la partie 24 qui se trouve en regard de l'orifice 7.

Ainsi, lorsque l'aiguille a fini sa pénétration, il y a peu de chance pour que l'orifice 7 soit obturé par un élément solide qui aurait été comprimé dans la chair et qui, par un mouvement élastique, aurait alors tendance à pénétrer dans cet orifice pour l'obturer.

Ainsi, avec une telle configuration d'aiguille, en injectant par exemple un liquide par l'entrée 5, celui-ci pourra s'écouler de façon aisée à l'intérieur de la chair par l'orifice 7.

## Revendications

1. Aiguille pour seringue, pour applications médicales, dentaires ou vétérinaires, ou analogues, constituée d'un tube (1) relativement fin et cylindrique comportant un canal central (3), ledit tube comportant à une extrémité (6) une pointe de pénétration, ledit canal débouchant sensiblement au niveau de cette pointe de pénétration, CARACTERISEE EN CE QUE ladite pointe de pénétration est constituée par une surface (9) unique, suivant un plan incliné (10) sur l'axe (2) du tube cylindrique formant ainsi un biseau, ledit canal (3) débouchant par au moins un orifice (7) sur la paroi latérale dudit tube cylindrique à un niveau situé sensiblement à proximité de ladite surface (9) et sur une génératrice solide (12) dudit tube passant par la perpendiculaire (13) audit plan incliné (10) et élevée au point de concours (14) de l'axe (2) dudit tube cylindrique et dudit plan incliné.

2. Aiguille selon la revendication 1, CARACTERISEE EN CE QUE ledit canal (3) débouche par un deuxième orifice (8) dans ladite surface en biseau (9).

3. Aiguille selon l'une des revendications 1, et 2, CARACTERISEE EN CE QUE ledit canal débouche en outre sur la paroi latérale dudit tube (1), à proximité de ladite surface en biseau (9), par une pluralité d'autres orifices.

4. Aiguille selon l'une des revendications précédentes, CARACTERISEE EN CE QUE ladite surface en biseau (9) est une surface plane.

5. Aiguille selon l'une des revendications 1 à 3, CARACTERISEE EN CE QUE ladite surface en biseau (9) est une surface légèrement sphérique.

6. Aiguille selon la revendication 5, CARACTERISEE EN CE QUE ladite surface sphérique est une surface concave.

fig.1

fig.2

fig.3